Europäisches Patentamt

European Patent Office

Office européen des brevets

(19)

(11) Publication number: **0 132 537**
**A1**

(12) # EUROPEAN PATENT APPLICATION

(21) Application number: **84106070.0**

(22) Date of filing: **28.05.84**

(51) Int. Cl.⁴: **G 01 N 33/53**

(30) Priority: **31.05.83 JP 96650/83**
**31.05.83 JP 96651/83**

(43) Date of publication of application:
**13.02.85 Bulletin 85/7**

(84) Designated Contracting States:
**DE FR GB NL**

(71) Applicant: **DENKA SEIKEN CO., LTD.**
**12-1, Kabuto-cho Nihonbashi**
**Chuo-ku Tokyo(JP)**

(72) Inventor: **Motoda, Shosaku**
**12-2805, Higashihoridoori**
**Niigata-shi Niigata-ken(JP)**

(72) Inventor: **Sekine, Sakari**
**4090-1, Ooazakanose Kanose-machi**
**Higashikanbara-gun Niigata-ken(JP)**

(72) Inventor: **Imai, Satoru**
**Hei 1426, Ooazakariwa Muramatsu-machi**
**Nakakanbara-gun Niigata-ken(JP)**

(74) Representative: **Hrabal, Ulrich, Dr. Dipl.-Chem. et al,**
**Patentanwälte Popp, Sajda, v. Bülow, Hrabal & Partner**
**Widenmayerstrasse 48 Postfach 86 06 24**
**D-8000 München 86(DE)**

(54) Immunoassay.

(57) An immunoassay of the type which utilizes the hemolysis of red blood cells with an antibody bound on their surfaces as an indicator. Red blood cells on whose surfaces a first antibody specific to an antigen to be quantified is bound, an antigen to be quantified, a second antibody specific to the antigen to be quantified and produced by an animal of a species different from that of the animal producing the first antibody, and complement are reacted. The antigen is quantified from the degree hemolysis of the red blood cells caused by the activated complement. Alternatively, red blood cells on whose surfaces a first antibody specific to an animal immunoglobulin is bound, an antigen to be quantified, a second antibody specific to the antigen to be quantified and produced by an animal of the same species as that of the animal producing the immunoglobulin specific to the first antibody, and complement are reacted. The antigen is quantified from the degree of hemolysis of the red blood cells caused by the activated complement.

EP 0 132 537 A1

- 1 -

## Immunoassay

The present invention relates to an immunoassay. More particularly, the present invention relates to an immunoassay for quantitative analysis of an antigen by measuring a degree of hemolysis caused by the lysis of red blood cells through the reaction between an antigen-antibody complex and a complement.

In the medical field, the quantitative analysis of an antigen or other material with high reliability and simple operation has recently become extremely significant for the diagnosis of diseases.

Conventional types of immunoassay include (A) radioimmunoassay, (B) enzyme immunoassay, (C) reverse passive hemagglutination method, and (D) single radial immunodiffusion method. These immunoassays, however, have the following disadvantages.

Assay (A): This assay requires the use of a radioisotope, with the result that the equipment is rendered large and operations are complicated. Furthermore, it often requires washing operations.

Assay (B): Generally, this assay requires washing operations and a long period of time for analysis.

Assay (C): This assay requires a stepwise two-fold dilution operation on a specimen with a diluter, a dropwise addition of a diluting solution and other materials with a dropper, so that laborious operations are required. This assay also requires a long period of time for analysis. Further, it may produce inaccurate assay results because the amount of antigen is determined on the basis of the final value of the stepwise two-fold dilution.

Assay (D): This assay requires a long period of time for analysis and sensitivity is not satisfactory.

Heretofore, the complement fixation test is well known as a method of measuring a trace amount of an antigen or an antibody. The complement fixation test consists of two steps. The first step involves the reaction of a mixture of an antibody, an antigen, and a predetermined amount of complement. This reaction allows the resulting antigen-antibody complex to be combined with the complement and, consequently, the complement is consumed. The second step is to add to the resulting reaction mixture sheep erythrocytes on which an antibody specific to the sheep erythrocytes is bound. The addition allows the complement to be activated by the antigen-antibody complex (a red blood cell membrane and an antibody thereto) on the surfaces of the sheep erythrocytes, leading to the hemolysis of the sheep erythrocytes by the activated complement. The degree of hemolysis depends on the amount of the complement consumed in the first step. That is, in the case where a lot of complement is consumed in the first step, i.e., where a lot of antigen-antibody complex is formed, the degree of hemolysis is rendered small because the amount of the complement remaining in the second step is small. Conversely, in instances where not so much of the complement is consumed in the first step, i.e., where not so much of the antigen-antibody complex is formed, a remarkably high degree of hemolysis occurs because a large amount of complement remains in the second step. It is, accordingly, feasible to determine the amount of an antigen or an antibody reacting in the first step by measuring the degree of hemolysis in the second step by a certain method while the amount of the complement is kept at a constant level.

However, the conventional complement fixation test requires the inactivation of the complement in the test sample by treating the test serum at 56°C for 30 minutes in order to eliminate the effect of the complement in the test serum. It is further to be noted that the conventional complement fixation test requires laborious operations, such as stepwise two-fold dilution of the test serum, and a long period of time for quantitative analysis.

It is, therefore, an object of the present invention to provide an immunoassay capable of rapid quantitative determination of an antigen by simple operations and with high sensitivity.

According to one aspect of the present invention, a suspension of red blood cells, on whose surfaces a first antibody specific to the antigen to be quantified is bound, is prepared. The red blood cell suspension is then mixed with an antigen to be quantified, a second antibody which is specific to the antigen to be quantified and which is produced by an animal of a different species from that which produces the first antibody, and complement. By so doing, the constituents of the resulting mixture react with each other. As a result, the red blood cells are lysed by the complement activated by the formed antigen-antibody complex. The degree of hemolysis is then observed, for example, by measuring an absorbance of the mixture. With quantitative analysis carried out under constant conditions, such as constant amounts of the first and second antibodies as well as the amount of the red blood cells, the degree of hemolysis or absorbance of the mixture is in proportion to the amount of the antigen. It is thus possible to quantify the antigen from the observed degree of hemolysis.

According to another aspect of the present invention, a suspension of red blood cells, on whose surfaces a first antibody specific to an immunoglobulin from a certain animal is bound, is prepared. The red blood cell suspension is then mixed with an antigen to be quantified, a second antibody specific to the antigen to be quantified and produced by an animal of the same species which produces the immunogloblin specific to the first antibody, and complement. On doing this, the constituents of the mixture react with each other to lyse the red blood cells by the complement activated by the formed antigen-antibody complex. The amount of the antigen is determined by measuring the degree of hemolysis in the same manner as in the first aspect of the present invention.

By the immunoassay according to the present invention, an antigen can be quantified quickly by simple operations and with good sensitivity.

This invention can be more fully understood from the following detailed description when taken in conjunction with the accompanying drawings, in which:

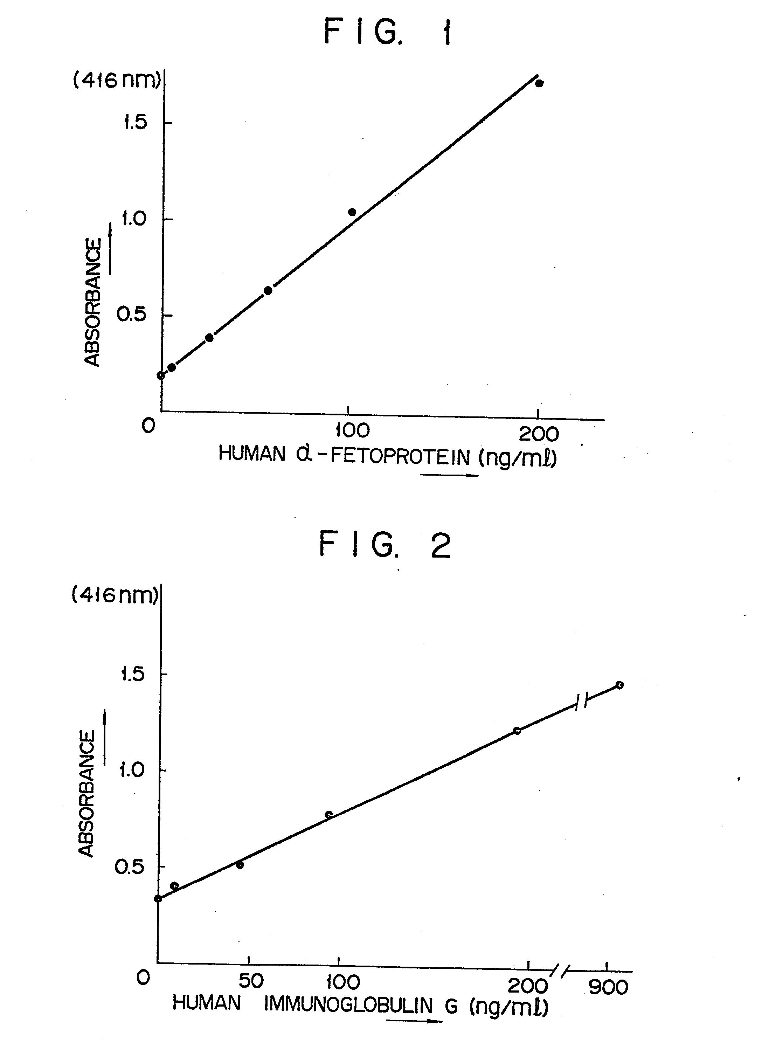

Figs. 1 through 4 respectively show a calibration curve obtained by the immunoassay according to the present invention.

According to one aspect of the present invention, a suspension of red blood cells on the surfaces of which a first antibody specific to an antigen to be quantified is bound, is first provided.

The antigen to be quantified may include, for example, α-fetoprotein (α-FP), hepatitis B surface antigen (HBs), immunoglobulin G, immunoglobulin M, myoglobin, ferritin, and C-reactive protein (CRP). As the immunoassay according to the present invention permits the quantitative analysis of a variety of antigens, it is to be understood that the antigens to be quantified are not intended to be limited to those enumerated above.

The first antibody specific to the antigen to be quantified may be produced by administering the antigen to a suitable animal, collecting blood from the animal after a suitable period of time, and treating the resulting serum in a conventional manner. It is to be understood that the kind of animal is not limited to any particular one. As such antibodies specific to various antigens are commercially available, these commercially available antibodies can also be employed.

Red blood cells from any animal can be employed for the method according to the present invention. It is preferred, however, to use sheep red blood cells which can be easily lysed by the complement activity, because they permit higher sensitivity in quantitative analysis.

The techniques for binding the antibody to the surfaces of red blood cells are well known in the pertinent field. For instance, Molinaro et al., U.S. Patent No. 4,130,634, issued on December 19, 1978, which is hereby incorporated by reference, discloses various such methods. More specifically, the mixing of a red blood cell suspension with an antibody in the presence of tannic acid, chromium chloride or a water-soluble carbodiamide permits the antibody to be bound to the surfaces of the red blood cells. Red blood cells may be suspended in physiological saline or any other acceptable physiological buffer solution.

As described above, the suspension of the red blood cells with the first antibody specific to the antigen to be quantified and bound to the surfaces thereof is prepared.

The next step involves the mixing of the following: (a) the red blood cell suspension prepared as stated above, (b) the antigen to be quantified, (c) a second antibody specific to the antigen to be quantified and produced by an animal of a different species from that producing the first antibody, and (d) complememt.

The second antibody which is specific to the antigen to be quantified and which is produced by the animal of a different species from that producing the first antibody may be obtained by administering the antigen to be quantified to an animal of a different species from that from which the first antibody was collected, collecting blood from the animal, and then treating the blood in a conventional manner. The most critical requirement here is that the first and the second antibody be produced by animals of different species. For instance, in the case where the first antibody is produced by a goat, a rabbit may be chosen as an animal for producing the second antibody. The difference in the species of animals producing the first antibody and the second antibody, as herein described, permits the immunoassay to be carried out even if the four reagents (a), (b), (c), and (d) as described above are simultaneously mixed together, as will be apparent from the working examples, thus reducing the period of time required for the immunoassay. In the case where first and second antibodies from an animal of the same species are used, a longer period of time is required because the reagent (a) must first react with the reagent (b), and then the reagents (c) and (d) are added thereto. It is preferred to use a rabbit or guinea pig as an animal for producing the second antibody because the sensitivity of the quantitative analysis is then increased. As antibodies specific to various antigens produced from various animals are commercially available, such commercially available antibodies may be used as both the first and the second antibodies.

The complement to be used for the immunoassay according to the present invention may be complement from any animal, and complement from a guinea pig which has a high complement activity is preferred. Guinea pig serum, diluted appropriately, may be used as it is as complement.

As described above, the second step of the immunoassay according to the present invention involves the mixing of the four reagents (a), (b), (c), and (d), and reacting the constituents of the mixture with each other. The mixing step may be carried out by mixing all four reagents at substantially the same time or by simultaneously mixing the three reagents (a), (b), and (c) and then adding the reagent (d) thereto. Needless to say, it is further feasible to react the reagents (a) and (b) first and then to add the reagents (c) and (d) thereto, as in the case where first and second antibodies from an animal of the same species are employed.

To accomplish the reaction, the resulting mixture is then incubated under suitable conditions. The conditions for incubation may be from 25°C to 45°C, preferably from 35°C to 40°C, and from 10 minutes to 120 minutes, preferably from 30 minutes to 60 minutes.

Red blood cells are lysed by the reaction. This hemolysis is considered to be caused by the complement activated by the antigen-antibody complex that is formed by the binding of the antigen to be quantified to the first antibody present on the surfaces of the red blood cells and then the second antibody to the antigen.

The degree of hemolysis is then observed by, for example, measuring the absorbance of the resulting mixture. The greater the degree of hemolysis, the larger the observed absorbance of the mixture because of the hemoglobin released from the lysed red blood cells. If the immunoassay is conducted several times under identical conditions in terms of the kind and concentration of the red blood cell suspension, the amount of the second antibody and other parameters, then the degree of hemolysis, e.g., the absorbance of the mixture, is in proportion to the amount of the antigen to be quantified. Accordingly, if a calibration curve is once prepared, it is possible to determine the amount of the antigen to be quantified from the degree of hemolysis observed, using the calibration curve.
Techniques of preparing a calibration curve are well
known in the immunoassay field.  A calibration curve can
be prepared by measuring the degree of hemolysis, e.g.,
absorbance for mixtures containing different predetermined amounts of an antigen, and by plotting the amounts
of the antigen along the axis of abscissa and the
corresponding absorbances along the axis of ordinate.

According to another aspect of the present
invention, a suspension of red blood cells having the
first antibody specific to an immunoglobulin of a
certain animal bound on their surfaces is prepared.
Preferable animals include rabbits and guinea pigs.  The
animal immunoglobulins may be one in a crude form
manufactured by removing proteins such as albumin from
an animal serum by salting it out with ammonium sulfate,
or one in a purer form obtainable by purifying the crude
one.  The preferred immunoglobulin is immunoglobulin G
(IgG).  A variety of antibodies specific to
immunoglobulins are commercially available, and such
commercially available anti-immunoglobulin antibodies
can be preferably employed.

The red blood cells to be used and the binding of
the anti-immunoglobulin antibody to the surfaces of red
blood cells are the same as described above.

The next step involves the mixing of the following
four reagents and a reaction thereamong: (a) the red
blood cell suspension described above; (b) the antigen
to be quantified; (c) the second antibody specific to
the antigen to be quantified and produced by an animal
of the same species as produces the immunoglobulin
specific to the first antibody (i.e., anti-
immunoglobulin antibody); and (d) complement.

The antigen to be quantified is as described above.

The second antibody, which is specific to the
antigen to be quantified and is produced by an animal of
the same species as produces the immunoglobulin specific to the first antibody (i.e., anti-immunoglobulin antibody), can be prepared by administering the antigen to be quantified to the animal of the same species as produces the immunoglobulin specific to the first antibody, collecting blood from the animal after a suitable period of time, and then treating the resulting serum in a conventional manner. As described hereinabove, different kinds of these antibodies are commercially available, and such commercially available antibodies can be preferably employed.

The complement to be used is as described above.

The mixing of the four reagents (a), (b), (c), and (d) may be conducted at substantially the same time, or by first mixing the reagent (a) with the reagent (b) and then adding the reagents (c) and (d) thereto. The former method is preferred, since it shortens the period of time required for the immunoassay.

The conditions for incubation of the mixture are the same as described above.

The mixing causes the hemolysis of the red blood cells. This hemolysis is considered to be caused by the complement activated by the antigen-antibody complex formed by binding the antigen to be quantified to the second antibody (this is an immunogloblin so that this is also an antigen to the anti-immunoglobulin antibody) bound to the first antibody on the surfaces of the red blood cells.

The antigen to be quantified may be quantified by observing a degree of hemolysis as described hereinabove.

In the immunoassay according to this aspect of the present invention, what is bound to the surfaces of the red blood cells is an antibody specific to an immunoglobulin (the second antibody), not an antibody specific to a particular antigen. It is thus convenient since the red blood cells with the anti-immunoglobulin antibody bound thereto can be used for quantifying

- 10 -

0132537

various antigens.

Example 1

Quantitative Determination of Human α-Fetoprotein

One part by volume of sheep red blood cell precipitate washed with physiological saline, 2 parts by volume of 0.4 mg/ml chromium chloride ($CrCl_3 \cdot 6H_2O$) solution in physiological saline, and 2 parts by volume of a solution of anti-human α-fetoprotein goat antibody (commercially available from MBL (KK) Igaku Seibutsugaku Kenkyusho, 3-5-10, Marunouchi, Naka-ku, Nagoya, Japan, sold under Code No. 157, obtained by fractionating a serum of a goat immuned with human α-fetoprotein by salting out with 50% ammonium sulfate and then with 33% ammonium sulfate and dialyzing the fraction against physiological saline) in physiological saline (protein content: 400 µg/ml) were mixed and allowed to react at room temperature for 30 minutes. The red blood cells were washed with physiological saline and then suspended in physiological saline to give a suspension containing 8% by volume of antibody-bound red blood cells.

Fifty µl of the antibody-bound red blood cell suspension, a sample solution of a different concentration of human α-fetoprotein (produced by Behring Institute, Marburg, W. Germany; Tradename: α-Fetoprotein Standard Serum (human)) in 50 µl of physiological saline, and a solution of anti-human α-fetoprotein rabbit antibody (product obtained by dialyzing the crude anti-human α-fetoprotein antibody manufactured by DAKOPATTS a/s, 22, Guldborg vej, Post box 1404, DK-2000 Copenhagen F, Denmark, under Code No. A008 against physiological saline) in 50 µl of physiological saline (protein content: 400 µg/ml) were mixed and allowed to react at 37°C for 10 minutes, and the resulting reaction mixture was mixed with 3.0 ml of a 100-fold dilution of guinea pig serum with Veronal buffer as complement. The mixture was allowed to react at 37°C for 30 minutes, and then centrifuged at 2,000 r.p.m. for 5 minutes. The resultant supernatant was measured for absorbance at 416 nm with a spectrophotometer (Model 100-21, Hitachi, Ltd.).

Fig. 1 shows a calibration curve formed by plotting the concentration of the human α-fetoprotein in the samples along the axis of abscissa, and the corresponding absorbance along the axis of ordinate. As will be apparent from Fig. 1, a proportional relationship exists between the α-fetoprotein concentration and the absorbance measured. It is thus possible to quantify an unknown amount of human α-fetoprotein using the calibration curve.

Example 2

Quantitative Determination of Human Immunoglobulin G

One part by volume of sheep red blood cell precipitate washed with physiological saline, 2 parts by volume of chromium chloride ($CrCl_3 \cdot 6H_2O$) in physiological saline (0.4 mg/mℓ), and 2 parts by volume of anti-human immunoglobulin G (specific to the γ-chain) sheep antibody (a product obtained by dialyzing the crude anti-human immunoglobulin G sheep antibody, manufactured by Cappel Laboratories Inc., Cochranville, U.S.A., under Code No. 0201-0124 against physiological saline) in physiological saline were mixed and allowed to react at room temperature for 30 minutes. After the red blood cells were washed with physiological saline, they were suspended in physiological saline to give a suspension containing 8% by volume of the antibody-bound red blood cells.

Fifty μℓ of the antibody-bound red blood cell suspension, a sample of a different concentration of human immunoglobulin G (manufactured by The Green Cross Corporation, 1-15-1, Imabashi, Higashi-ku, Osaka, Japan) in 50 μℓ of physiological saline, and anti-human immunoglobulin G (specific to the γ-chain) rabbit antibody (Code No. 0201-0122, manufactured by Cappel Laboratories Inc.) in 50 μℓ of physiological saline were mixed and allowed to react at 37°C for 10 minutes. This reaction mixture was then mixed with 3.0 ml of a 100-fold dilution of guinea pig serum with Veronal buffer, and the mixture was reacted at 37°C for 30 minutes. The reaction mixture was then centrifuged in the same manner as in Example 1, and the resultant supernatant was measured for its absorbance.

Fig. 2 shows a calibration curve formed by plotting the concentration of the human immunoglobulin G in the samples tested along the axis of abscissa, and plotting the corresponding absorbance observed along the axis of ordinate. It is found that a proportional relationship exists between the antigen concentration in the test samples and the absorbance. It is thus possible to quantify an unknown amount of human immunoglobulin G using the calibration curve.

Example 3

This example is to show the necessity for the second antibody. It is to be noted that, in the following examples, the reagents and their concentrations are the same as used in Example 1 or 2 unless otherwise stated.

(i) Fifty μl of a suspension of red blood cells with an anti-human α-fetoprotein goat antibody bound thereto, prepared in the same manner as in Example 1, and 50 μl of a solution of human α-fetoprotein (200 ng/ml) in physiological saline were mixed and the mixture was incubated at 37°C for 5 minutes. Fifty μl of anti-human α-fetoprotein rabbit antibody were added to this mixture, and the mixture was incubated at 37°C for 5 minutes. 3.0 ml of a 100-fold dilution of guinea pig serum with Veronal buffer was added to this mixture, and the mixture was reacted at 37°C for 30 minutes. The reaction mixture was then centrifuged, and the resultant supernatant was measured for absorbance in the same manner as in Example 1.

(ii) As a control, the procedures described in item (i) above were followed with the exception that physiological saline was used in place of the anti-human α-fetoprotein rabbit antibody.

(iii) As a control, the procedures described in item (i) above were followed with the exception that physiological saline was used in place of both the human α-fetoprotein and the anti-human α-fetoprotein rabbit antibody, respectively.

The results are shown in Table 1 below.

Table 1

| Operations | Absorbance |
|------------|------------|
| (i) | 1.630 |
| (ii) | 0.150 |
| (iii) | 0.145 |

From this table, it can be seen that the addition of the second antibody (Operation (i)) apparently caused hemolysis (absorbance of 1.630), whereas the non-addition of the second antibody (Operation (ii)) showed a virtually identical absorbance as in the case of not adding both the second antibody and antigen (Operation (iii)). This means that not adding the second antibody causes virtually no hemolysis.

Example 4

This example is to show the advantages of using different species of animals for producing the first and the second antibody.

(A-i) A suspension of red blood cells with anti-human α-fetoprotein goat antibody bound thereto was prepared in the same manner as in Example 1. Fifty µℓ of the suspension was mixed with 50 µℓ of human α-fetoprotein in physiological saline (500 ng/mℓ), and 50 µℓ of an anti-human α-fetoprotein rabbit antibody in physiological saline and then incubated at 37°C for 10 minutes. The mixture was then mixed with 3.0 mℓ of a 100-fold dilution of guinea pig serum with Veronal buffer, and reacted at 37°C for 30 minutes. The reaction mixture was centrifuged and the resultant supernatant was measured for absorbance in the same manner as in Example 1.

(A-ii)  The above procedures of Operation (A-i) were followed with the exception that physiological saline was used in place of a solution of human α-fetoprotein in physiological saline.

(B-i)  A suspension of red blood cells with an anti-human α-fetoprotein rabbit antibody bound thereto was prepared in the same manner as in Example 1 with the exception that the anti-human α-fetoprotein rabbit antibody was used in place of the anti-human α-fetoprotein goat antibody.  This suspension was treated in the same manner as in Operation (A-i) above.

(B-ii)  The above procedures of Operation (A-i) were followed with the exception that physiological saline was employed in place of a solution of the human α-fetoprotein in physiological saline.

Table 2

| Operations | Absorbance |
|------------|------------|
| (A-i)  | 1.705 |
| (A-ii) | 0.328 |
| (B-i)  | 0.340 |
| (B-ii) | 0.320 |

As shown in the Table 2, the hemolysis is caused apparently when the first and the second antibody were produced by animals of different species, thus enabling human α-fetoprotein to be quantified (Operation (A-i)). On the other hand, it is found that the absorbance measured in the case of using first and second antibodies from the same species of animal (Operation (B-i)) is almost the same as that in the case where no antigen is added (Operation (B-ii)), thus causing virtually no hemolysis. In the case where no antigen was added, the absorbance in Operation (A-ii) is almost the same as that in Operation (B-ii). Therefore, it can be seen that the difference in absorbance in Operations (A-i) and (B-i) were brought about by the fact that in (A-i), the first and the second antibodies were produced by the animals of the same species, while in (B-i) they were produced by animals of different species.

As will be apparent from this Example, the immunoassay according to the present invention enables the antigen to be quantified even where a suspension of red blood cells with the first antibody bound thereto, the antigen and the second antibody are simultaneously mixed together. On the other hand, in the case where the first and the second antibodies are produced by the same species of animals, the antigen cannot be quantified if the above three reagents are mixed simultaneously.

Example 5

Quantitative Determination of Human α-Fetoprotein

One part by volume of sheep red blood cell precipitate washed with physiological saline, 2 parts by volume of chromium chloride $(CrCl_3 \cdot 6H_2O)$ in physiological saline (0.4 mg/mℓ), and 2 parts by volume of anti-rabbit immunoglobulin G goat antibody (a product obtained by dialyzing the crude anti-rabbit immunoglobulin G goat antibody manufactured by Cappel Laboratories Inc. under Code No. 0212-0121, against physiological saline) in physiological saline (protein content: 400 µg/mℓ) were mixed and allowed to react at room temperature for 30 minutes. The red blood cells were washed with physiological saline and then suspended in physiological saline to give a suspension containing 8% by volume of the antibody-bound red blood cells.

Fifty µℓ of this antibody-bound red blood cell suspension was mixed with 50 µℓ of an anti-human

α-fetoprotein rabbit antibody in physiological saline, and 50 μl of a solution of a varied concentration of human α-fetoprotein in physiological saline, and the mixture was incubated at 37°C for 10 minutes. 3.0 ml of a 100-fold dilution of guinea pig serum with Veronal buffer were added to the mixture, and the mixture was reacted at 37°C for 30 minutes. The reaction mixture was then centrifuged, and the resultant supernatant was measured for absorbance in the same manner as in Example 1.

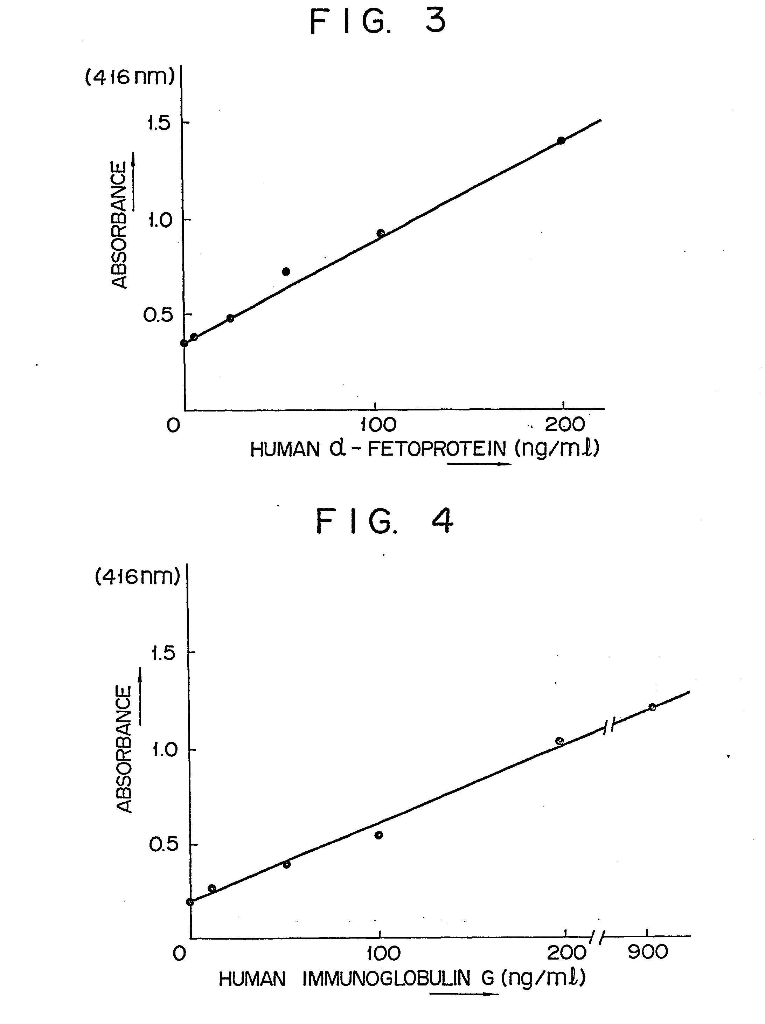

Fig. 3 shows a calibration curve formed by plotting the human α-fetoprotein concentration in the test samples along the axis of abscissa and the observed absorbance along the axis of ordinate. As shown in Fig. 3, a proportional relationship exists between the human α-fetoprotein concentration and the absorbance. It is thus to be noted that an unknown amount of human α-fetoprotein may be quantified using the calibration curve.

Example 6

Quantitative Determination of Human Immunoglobulin G

A suspension of red blood cells with an anti-rabbit immunoglobulin G goat antibody was prepared in the same manner as in Example 5. Fifty μl of this suspension was mixed with 50 μl of an anti-human immunoglobulin G (specific to the γ-chain) rabbit antibody (protein content: 200 μg/ml) in physiological saline, and a sample of human immunoglobulin G of varied concentration in physiological saline, and the mixture was incubated at 37°C for 10 minutes. 3.0 ml of a 100-fold diluted guinea pig serum with Veronal buffer was then added to the mixture, and the mixture was reacted at 37°C for 30 minutes. The reaction mixture was centrifuged in the same manner as in Example 1 and the supernatant was measured for absorbance.

Fig. 4 shows a calibration curve formed by plotting the human immunoglobulin G concentration in the test samples along the axis of abscissa and the observed absorbance along the axis of ordinate. It is found from this calibration curve that a proportional relationship exists between the antigen concentration in the test sample and the absorbance. It is thus to be noted that an unknown amount of human immunoglobulin G may be quantified using the calibration curve.

# POPP, SAJDA, v. BÜLOW, HRABAL & PARTNER

Patentanwälte · European Patent Attorneys
München · Bremen*

**0132537**

-1-

Popp, Sajda, v. Bülow, Hrabal & Partner, Postfach 86 06 24, D-8000 München 86

**Dr. Eugen Popp** Dipl.-Ing., Dipl.-Wirtsch.-Ing.
**Wolf E. Sajda** Dipl.-Phys.
**Dr. Tam v. Bülow** Dipl.-Ing., Dipl.-Wirtsch.-Ing.
**Dr. Ulrich Hrabal** Dipl.-Chem.
**Erich Bolte** Dipl.-Ing.*

**BÜRO MÜNCHEN/MUNICH OFFICE:**
Widenmayerstraße 48
Postfach/P.O. Box 86 06 24
D-8000 München 86
Telefon: (089) 22 26 31
Telex: 5 213 222 epo d
Telekopierer: (089) 22 17 21

Applicant:

DENKA SEIKEN CO., LTD.

M/SUS-15-EP

| Ihr Zeichen<br>Your ref | Ihr Schreiben vom<br>Your letter of | Unser Zeichen<br>Our ref. | Datum<br>Date |
|---|---|---|---|
| | | | May 28, 1984 |

Immunoassay

Claims:

1. An immunoassay comprising, in the order mentioned, the steps of:

   providing a suspension of red blood cells on whose surfaces a first antibody specific to an antigen to be quantified is bound;

   mixing the red blood cell suspension, the antigen to be quantified, a second antibody specific to the antigen to be quantified and produced by an animal of a species different from that of the animal which produces the first antibody, and complement and allowing these reagents to react, thereby lysing the red blood cells; and

   determining the degree of the hemolysis caused, thereby quantifying the antigen.

2. The immunoassay according to claim 1, characterized in that said third step is carried out by measuring the absorbance of the mixture obtained in said second step.

3. The immunoassay according to claim 1 or 2, characterized in that the animal which produces the second antibody is a rabbit or guinea pig.

4. The immunoassay according to claim 1, 2 or 3, characterized in that, in said second step, the red blood cell suspension, the second antibody, and the antigen are mixed at substantially the same time.

5. The immunoassay according to claim 1, 2, 3 or 4, characterized in that the reaction in the said second step is carried out at 25°C to 45°C for 10 minutes to 120 minutes.

6. The immunoassay according to claim 1 or claims 2 to 5, characterized in that the red blood cells are sheep red blood cells.

7. An immunoassay comprising, in the order mentioned, the steps of:

providing a suspension of red blood cells with a first antibody specific to an animal immunoglobulin bound on the surfaces thereof;

mixing the red blood cell suspension, a second antibody specific to an antigen to be quantified and produced by an animal of the same species as that of the animal which produces the animal immunoglobulin, the antigen to be quantified, and complement, and allowing these reagents to react, thereby lysing the red blood cells; and

determining the degree of the hemolysis caused, thereby quantifying the antigen.

8. The immunoassay according to claim 7, characterized in that said third step is carried out by measuring the absorbance of the mixture obtained in said second step.

9. The immunoassay according to claim 7 or 8, characterized in that the animal producing the first and the second antibodies is a rabbit or a guinea pig.

10. The immunoassay according to claim 7, 8 or 9, characterized in that the immunoglobulin is immunoglobulin G.

11. The immunoassay according to claim 7, 8, 9 or 10, characterized in that the red blood cells are sheep red blood cells.

12. The immunoassay according to claim 7 or claims 8 to 11, characterized in that the second step is carried out at 25°C to 45°C for 10 minutes to 120 minutes.

1/2

0132537

# F I G. 1

(416 nm)

ABSORBANCE

1.5

1.0

0.5

0   100   200

HUMAN α-FETOPROTEIN (ng/mℓ)

# F I G. 2

(416 nm)

ABSORBANCE

1.5

1.0

0.5

0   50   100   200   900

HUMAN IMMUNOGLOBULIN G (ng/mℓ)

2/2    0132537

FIG. 3

FIG. 4

0132537

Application number

EP  84 10 6070

European Patent Office

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (Int Cl 4) |
|---|---|---|---|
| D,X | US-A-4 130 634  (G.A. MOLINARO et al.)<br>* whole document * | 1-12 | G 01 N  33/53 |
| | --- | | |
| X | NATURE, vol. 248, 5th April 1974, pages 515-517, MacMilan Journals Ltd., Basingstoke, GB; G.A. MOLINARO et al.: "Antibody coated erythrocytes as a manifold probe for antigens"<br>* whole article * | 1-12 | |
| | ----- | | |

TECHNICAL FIELDS SEARCHED (Int. Cl.4)

G 01 N

The present search report has been drawn up for all claims

| Place of search | Date of completion of the search | Examiner |
|---|---|---|
| THE HAGUE | 14-09-1984 | GRIFFITH G. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82